# EUROPÄISCHE PATENTSCHRIFT

(11) **EP 0 363 835 B1**
(45) Veröffentlichungstag und Bekanntmachung des Hinweises auf die Patenterteilung: **16.06.1993**
(21) Anmeldenummer: 89118565.4
(22) Anmeldetag: 06.10.1989
(51) Int. Cl.: A61K 39/39, A61K 33/26, A61K 33/30

(54) **Verwendung von Zink- oder Eisenhydroxid zur Adjuvierung von Antigenlösungen und auf diese Weise adjuvierte Antigenlösungen**
Use of zinc or iron hydroxide in the adjuvation of antigen solutions, and antigen solutions adjuvated in this manner
Application de l'hydroxyde de zinc ou de fer pour comme adjuvant des solutions d'antigènes et solutions d'antigènes préparées de cette manière

(30) Priorität: 12.10.1988 DE 3834729
(43) Veröffentlichungstag der Anmeldung: 18.04.1990
(73) Patentinhaber: BEHRINGWERKE Aktiengesellschaft, 35001 Marburg (DE)
(72) Erfinder: Bernhardt, Dieter, Dr., D-3553 Cölbe (DE)

(56) Entgegenhaltungen:
- EP-A- 0 108 316
- GB-A- 1 189 340

## Beschreibung

Die Erfindung betrifft die Verwendung von Zink- oder Eisen-Hydroxidgel und gegebenenfalls einem Lecithin zur Adjuvierung von Antigenlösungen sowie auf diese Weise adjuvierte Antigenlösungen.

Viele Antigene sind sowenig immunogen, daß sie nach einmaliger Injektion in ein Tier oder einen Menschen keine meßbare oder nur geringe Immunantwort auslösen. Um die Immunantwort des Organismus auf einen Antigenreiz zu verstärken, werden deshalb dem Antigen Adjuvantien zugesetzt. Die meisten inaktivierten Virus- und Bakterienvaccinen enthalten aus diesem Grund Adjuvantien. In den erwähnten Virus- und Bakterienimpfstoffen benutzt man überwiegend Al(OH)₃ und AlPO₄, einzeln oder in Kombination, Pflanzenöle oder aus Erdölfraktionen gewonnene Mineralöle, sogenannte medizinisch pharmazeutische Weißöle nicht exakt definierter Zusammensetzung. Freundskomplettes und inkomplettes Adjuvans, bestehend aus dem Mineralöl ^{®}Bayol F mit und ohne Extrakt von Mycobakterien, wird vorwiegend in experimentellen Vaccinen eingesetzt.

Diese Adjuvantien können aber neben Lokalreaktionen auch systemische Nebenwirkungen entfalten.

Neben der lokalen und allgemeinen Verträglichkeit von Adjuvantien sind von vitalem Interesse:
1. ihre immunologischen Wirkmechanismen, die sie induzieren;
2. ihre Pharmakokinetik (Bioabbaubarkeit).

Zu 1: So ist allgemein bekannt, daß die mineralischen Adjuvantien (Al(OH)₃, AlPO₄) vorwiegend nur eine humorale Immunantwort induzieren, während die zelluläre Immunität, die bei vielen Virusinfektionen eine dominierende Rolle spielt, nur geringfügig oder gar nicht stimuliert wird.

Anders verhalten sich die Mineralöle und besonders Freund's Adjuvans, die bekanntermaßen sowohl die zelluläre als auch die humorale Immunantwort stimulieren.

Zu 2: Die bisher gebräuchlichen Adjuvantien verbleiben größtenteils an der Injektionsstelle oder werden abtransportiert und in anderen Organen des Organismus angereichert, wo sie ihre immunologische und toxische Wirkung entfalten, d. h. ein Abbau bzw. eine Ausscheidung findet nicht oder nur sehr verzögert statt.
Anders verhalten sich die erfindungsgemäßen Adjuvantien Zinkhydroxid-Gel, Eisenhydroxid-Gel und Lecithin. Diese unterliegen im Organismus dem Stoffwechsel. Sie sind daher weniger toxisch.

In EP-A-0 108 316 (deutsche Offenlegungsschrift 32 41 113) wurde vorgeschlagen, neben anderen Stoffen Verbindungen des Zinks, und zwar Zinksalze als Zuschlagstoffe zu Vaccinen zu verwenden.

In Vorversuchen an Mäusen und Meerschweinchen wurde jedoch festgestellt, daß Zn-Salze wie Zinkchlorid, Zinksulfat und Zinkacetat oder Eisensalze wie Eisen-III-Chlorid in wässriger Lösung lokal recht unverträglich sind und keinerlei meßbare Adjuvanswirkung aufweisen.

Überraschenderweise wurde aber nun gefunden, daß Zinkhydroxid- und Eisenhydroxid-Gele, die man aus den entsprechenden Salzlösungen gewinnen kann, folgende Eigenschaften aufweisen:
1. Zinkhydroxid- und Eisenhydroxid-Gele besitzen sehr gute adsorbierende Eigenschaften (Virus und Protein)
2. Zinkhydroxid- und Eisenhydroxid-Gele besitzen sehr gute adjuvierende Eigenschaften
3. Zinkhydroxid-Gel induziert sowohl die humorale wie auch zelluläre Immunität
4. Eisenhydroxid-Gel induziert vorwiegend die humorale Immunität
5. Zinkhydroxid- und Eisenhydroxid-Gele besitzen im Vergleich zu den bisher üblichen Adjuvantien eine recht gute lokale und allgemeine Verträglichkeit
6. Durch die Zugabe eines Lecithins zu Zinkhydroxid- und Eisenhydroxid-Gel wird die adjuvierende Wirkung der Hydroxid-Gele noch verstärkt, die lokale Verträglichkeit noch gesteigert und die Galenik der Vaccinen noch verbessert.

Verfahrensbeschreibung der Herstellung von Zinkhydroxid-Gel, Eisenhydroxid-Gel und Lecithin 99-Suspension nach an sich bekannten Verfahren:

### Zinkhydroxid-Gel

1. Ausgehend von ZnCl₂, Zn-Acetat x 2H₂O, ZnSO₄ x 7H₂O-Herstellung einer 0,1 M-Lösung in Aqua dest.
2. Ausgehend von Zn-Carbonathydroxid-Herstellung einer 0,1 M-Lösung in Aqua dest. durch Zugabe von 5 N HCl.
3. Sterilfiltration der Zn-Salzlösung (0,2µ Membranfilter)
4. Unter sterilen Bedingungen und unter Rühren erfolgt die Zugabe von 10 N NaOH oder 10 N KOH bis ein pH von 6,0 - 7,8 erreicht ist.
5. Das ausgefallene Zinkhydroxid-Gel kann durch ^{®}ultraturrax-Behandlung weiter homogenisiert werden.

Die hier als Ausgang benutzten Zn-Salze sind nur beispielhaft. Auch aus den anderen Zn-Salzen (auch Zn-Oxid) können nach den beschriebenen Verfahren ebenfalls leicht Zinkhydroxid-Gele hergestellt werden. Ebenso ist es möglich, ein Zinkhydroxid-Gel unter pH-Kontrolle direkt in einer Antigensuspension herzustellen. Arbeitet man nicht mit sterilen Zn-Salz-Lösungen, sondern unter unsterilen Bedingungen, kann das Gel 20 min bei 120° C autoklaviert werden.

### Eisenhydroxid-Gel:

Bei der Herstellung von Eisenhydroxid-Gel wurde in der analogen Weise zu der Zinkhydroxid-Gel-Herstellung gearbeitet. Als Ausgang diente eine 0,25 M FeCl₃-Salzlösung in Aqua dest.

### Herstellung einer 20%igen Lecithin 99-Suspension

1. 20 g Lecithin werden in 100 ml PBS, pH 7,2 suspendiert;
2. Autoklavieren der Suspension 20 min bei 120° C;
3. Nach Abkühlen wird die Suspension homogenisiert;
4. pH-Einstellung der Suspension mit 10 N NaOH auf 7,0 - 7,8.

Diese Gele und gegebenenfalls Lecithin-Suspension werden einer Antigenlösung in folgenden Mengen zugesetzt:

| | |
|---|---|
| Zinkhydroxydgel | 1 - 45 % |
| Eisenhydroxydgel | 1 - 45 % |
| Lecithin-Suspension | 1 - 25 % |

Als Antigene kommen Virus-, Bakterien-, Zell-, Peptid-und Protein-Antigene in Frage.

Die folgenden Beispiele erläutern die Vorteile der erfindungsgemäßen immunstimulatoren/Adjuvantien.

### Beispiele

### Beispiel 1:

Aujeszky disease virus (AV) wurde in primären Schweinenierenzellkulturen vermehrt. Nachdem die Kulturen 100 %ig virusspezifisch zerstört worden waren, wurde das Virus geerntet und durch Zentrifugation und Filtration gereinigt. Hiernach erfolgte die AV-Inaktivierung mit Äthylenimin. Nach Sterilitäts- und Safetyprüfung wurden aus diesem inaktivierten AV-Antigen 6 Vaccinen hergestellt.

Die Zusammensetzung der Vaccinen ist nachfolgend in Tabelle 1 aufgeführt (Angaben in ml):

**Tabelle 1**

| Adjuvans/Antigen | **VACCINE** | | | | | |
|---|---|---|---|---|---|---|
| | A | B | C | D | E | F |
| Al(OH)₂ 2 %ig | 39,5 | | | | | |
| Saponin 10 %ig | 0,5 | | | | | |
| Zn-hydr. 0,1 M-Gel | | 40 | | | 20 | 17,5 |
| Fe-hydr. 0,25 M-Gel | | | 40 | | 20 | 17,5 |
| Lecith. 99* 20 %ig | | | | 40 | | 5,0 |
| AV-Antigen | 60,0 | 60 | 60 | 60 | 60 | 60,0 |
| Gesamt: | 100,0 | 100 | 100 | 100 | 100 | 100,0 |

| | | | | | | |
|---|---|---|---|---|---|---|
| *) Lecithin 99 (Fa. Paesel GmbH & Co. Borsigallee 6, 6000 Frankfurt | | | | | | |

Mit diesen 6 Vaccinen wurden jeweils 20 NMRI-Mäuse, 18 - 20 g schwer, mit 0,2 ml vacciniert. Vor der Vaccination und 4 Wochen nach Vaccination wurde den Tieren Blut zur Bestimmung der neutralisiertenden Antikörper gegen das Aujeszkyvirus entnommen. Zur Bestimmung der Schutzrate wurden die Mäuse 4 Wochen p. v. Mit 200 letalen Dosen Aujeszkyvirus belastet.

Die Resultate dieses Versuches sind nachfolgend in Tabelle 2 aufgeführt.

**Tabelle 2**

| Vaccine | neutral. Antikörpertiter vor Vaccination | neutral. Antikörpertiter 4 Wo. nach Vaccination | Schutzrate (PD₅₀) |
|---|---|---|---|
| A | kleiner als 1:2 | 1:12 | 2,0 |
| B | kleiner als 1:2 | 1:86 | 25,0 |
| C | kleiner als 1:2 | 1:60 | 4,0 |
| D | kleiner als 1:2 | 1:10 | 1,5 |
| E | kleiner als 1:2 | 1:110 | 30,0 |
| F | kleiner als 1:2 | 1:204 | 65,0 |

Aus diesem Versuch geht klar hervor, daß Zinkhydroxid und Eisenhydroxid höhere Antikörpertiter und Schutzraten gegen das Aujeszkyvirus stimulieren als die Standardadjuvanskombination in Vaccine A. Die höchsten Werte werden mit der neuen Adjuvanskombination (Vaccine F) Zinkhydroxid + Eisenhydroxid + Lecithin 99 erzielt.

### Beispiel 2:

Mit den Vaccinen aus Beispiel 1 wurden jeweils 3 Meerschweinchen, 450 - 500 g schwer, mit 0,2 ml intraplantar vacciniert. Die Tiere wurden täglich bis zum 28. Tag p.v. auf Schwellungen und sonstige pathologisch anatomische Veränderungen an dem vaccinierten Fuß untersucht, um die Unverträglichkeit der Vaccine festzustellen. Nach Schwere und Dauer der pathologisch anatomischen Veränderungen wurde ein Punktwert der Unverträglichkeit errechnet, der umso höher ist, je unverträglicher die Vaccine ist. Außerdem wurden den Tieren vor und 4 Wochen nach Vaccination Blutproben entnommen, um die neutralisierenden Antikörper gegen das Aujeszkyvirus zu bestimmen.

Die Resultate des Versuches sind in Tabelle 3 zusammengefaßt.

**Tabelle 3**

| Vaccine | Unverträglichkeitspunkte | neutral. Antikörpertiter vor Vaccination | neutral. Antikörpertiter 4 Wo nach Vaccination |
|---|---|---|---|
| A | 685 | kleiner als 1:2 | 1:8 |
| B | 212 | kleiner als 1:2 | 1:25 |
| C | 189 | kleiner als 1:2 | 1:14 |
| D | 98 | kleiner als 1:2 | 1:4 |
| E | 192 | kleiner als 1:2 | 1:34 |
| F | 140 | kleiner als 1:2 | 1:42 |

Dieser Versuch zeigt deutlich, daß die erfindungsgemäßen Adjuvantien, alle ganz erheblich verträglicher sind als das Standardadjuvans Al(OH)₃ + Saponin. Die Wirksamkeit von Zinkhydroxid- und Eisenhydroxid-Gel, gemessen als neutralisierender Antikörpertiter, ist gleichfalls besser als bei dem mitgeführten Standardadjuvans. Die beste Wirksamkeit bei guter Verträglichkeit wird bei der Kombination Zinkhydroxid + Eisenhydroxid + Lecithin 99 (Vaccine F) erreicht.

### Beispiel 3

Mit Vaccine A und F aus Beispiel 1 wurden jeweils 2 Schweine, 30 - 35 kg schwer, mit 2,0 ml s.c. vacciniert. Die Tiere wurden täglich bis zur 3. Woche p.v. auf Schwellungen und pathologisch anatomische Veränderungen von der Injektionsstelle untersucht, um den Punktwert der Unverträglichkeit beider Vaccinen zu ermitteln (s. Beispiel 2).

Zum Zeitpunkt der Vaccination, 1, 2 und 3 Wochen nach Vaccination wurde den Tieren Blut zur Bestimmung der neutralisierenden Antikörpertiter gegen das Aujeszkyvirus entnommen.

Die Resultate des Versuches sind in Tabelle 4 aufgeführt:

**Tabelle 4**

| Vaccine | Tier | Unverträglichkeitspunkte | nuetralis. Antikörpertiter | | | |
|---|---|---|---|---|---|---|
| | | | vor Vacc. | 1W.p.v. | 2W.p.v. | 3W.p.v. |
| A | 1 | 87 | *1:2 | *1:2 | *1:2 | 1:7 |
| | 2 | 96 | *1:2 | *1:2 | 1:4 | 1:12 |
| F | 3 | 19 | *1:2 | 1:5 | 1:17 | 1:32 |
| | 4 | 28 | *1:2 | 1:6 | 1:16 | 1:44 |

| | | | | | | |
|---|---|---|---|---|---|---|
| * bedeutet kleiner als | | | | | | |

Dieser Versuch zeigt klar, daß die Adjuvanskombination Zinkhydroxid + Eisenhydroxid + Lecithin 99 (Vaccine F) wesentlich verträglicher und wirksamer ist als das Standardadjuvans Al(OH)₃ + Saponin (Vaccine A).

### Beispiel 4

Parainfluenza 3-(PI₃) und IBR (infektiöses bovines Rhinotracheitis-Virus)-Virus wurden in primären Kälbernierenkulturen vermehrt. Nachdem die Kulturen 100%ig virusspezifisch zerstört worden waren, wurde das Virus geerntet und durch Zentrifugation und Filtration gereinigt. Danach erfolgte die Inaktivierung beider Virusarten mit Äthylenimin. Nach Sterilitäts- und Safetyprüfung wurden mit diesen inaktivierten Antigenen 2 Vaccinen hergestellt:

| | | |
|---|---|---|
| Vaccine A enthielt: | PI₃-Antigen | 4,5 ml |
| | IBR-Antigen | 4,5 ml |
| | Al(OH)₃, 2%ig | 1,0 ml |
| Vaccine B enthielt: | PI₃-Antigen | 4,5 ml |
| | IBR-Antigen | 4,5 mg |
| | Zinkhydroxid, 0,1 M | 0,4 ml |
| | Fe-Hydroxid, 0,25 M | 4,0 ml |
| | Lecithin 99, 20%ig | 0,2 ml |

Mit diesen Vaccinen wurden jeweils 3 Hunde mit 1,0 ml s.c. vacciniert und 4 Wochen danach mit gleicher Impfdosis revacciniert. Alle Tiere wurden täglich bis zum 28. Tag p.v. und bis zum 7 Tag revacc. auf Schwellungen und sonstige pathologisch anatomische Veränderungen an der Injektionsstelle zur Bewertung der Unverträglichkeit untersucht.

Vor Vaccination, 4 Wochen danach und 1 Woche nach der Revaccination wurden den Hunden zur Bestimmung der neutralisierenden Antikörper gegen PI₃- und IBR-Virus Blutproben entnommen.

Die Ergebnisse sind in Tabelle 5 aufgelistet.

**Tabelle 5**

| Vaccine | | Unverträgl.-keitspunkte | | neutralis. Antikörpertiter gegen | | | | | |
|---|---|---|---|---|---|---|---|---|---|
| | | | | PI₃ | IBR | PI₃ | IBR | PI₃ | IBR |
| | | 1.Vacc. | 2.Vacc. | vor Vacc. | | 4 Wo.p.v. | | 1 Wo.p.rev. | |
| A | 1 | 24 | 8+ | *1:2 | *1:2 | 64 | *1:2 | 3550 | *1:2 |
| | 2 | 21 | 6+ | *1:2 | *1:2 | 42 | *1:2 | 2344 | *1:2 |
| | 3 | 19 | 7+ | *1:2 | *1:2 | 18 | *1:2 | 1778 | 1:6 |
| B | 4 | 8 | 5 | *1:2 | *1:2 | 86 | 1:12 | 3090 | 1:85 |
| | 5 | 7 | 6 | *1:2 | *1:2 | 63 | 1:24 | 3090 | 1:97 |
| | 6 | 4 | 4 | *1:2 | *1:2 | 74 | 1:8 | 3550 | 1:43 |

| | | | | | | | | | |
|---|---|---|---|---|---|---|---|---|---|
| + Lokalreaktion bei Versuchsende noch vorhanden | | | | | | | | | |
| * bedeutet kleiner als | | | | | | | | | |

Dieser Versuch zeigt klar, daß bei Hunden die Adjuvanskombination der Vaccine B, Zinkhydroxid + Eisenhydroxid + Lecithin 99 wesentlich besser verträglich und wirksamer ist als das Standardadjuvans Al(OH)₃ in Vaccine A. Gegen das IBR-Virus waren bei allen 3 Hunden, die mit der Vaccine B geimpft worden waren, gute Antikörpertiter nachzuweisen. Von den Tieren, die mit Vaccine A geimpft wurden, wies nur das Tier 3 einen geringen Antikörpertiter auf.

## Patentansprüche (Patentansprüche für folgende(n) Vertragsstaat(en): AT, BE, CH, DE, FR, GB, IT, LI, LU, NL, SE)

1. Verwendung von Zinkhydroxidgel oder Eisenhydroxidgel und gegebenenfalls einem Lecithin zur Adjuvierung einer Antigenlösung.

2. Antigenlösung adjuviert nach Anspruch 1, dadurch gekennzeichnet, daß der Antigenlösung 1 - 45 % V/V Zinkhydroxidgel oder 1 - 45 % V/V Eisenhydroxidgel und gegebenenfalls 1 - 25 % V/V eines Lecithins zugegeben worden sind.

3. Verfahren zur Herstellung einer adjuvierten Antigenlösung, dadurch gekennzeichnet, daß einer Antigenlösung 1 - 45 % V/V Zinkhydroxidgel oder 1 - 45 % V/V Eisenhydroxidgel und gegebenenfalls 1 - 25 % V/V eines Lecithins zugegeben wird.

## Patentansprüche (Patentansprüche für folgende(n) Vertragsstaat(en): ES, GR)

1. Verfahren zur Herstellung einer adjuvierten Antigenlösung, dadurch gekennzeichnet, daß einer Antigenlösung 1-45 % V/V Zinkhydroxidgel oder 1-45 % V/V Eisenhydroxidgel und gegebenenfalls 1-25 % V/V eines Lecithins zugegeben wird.

## Claims (Claims for the following Contracting State(s): AT, BE, CH, DE, FR, GB, IT, LI, LU, NL, SE)

1. The use of zinc hydroxide gel or iron hydroxide gel and, where appropriate, a lecithin as adjuvant in an antigen solution.

2. An antigen solution containing an adjuvant as claimed in claim 1, wherein 1-45% V/V zinc hydroxide gel or 1-45% V/V iron hydroxide gel and, where appropriate, 1-25% V/V of a lecithin have been added to the antigen solution.

3. A process for the preparation of an antigen solution containing adjuvant, which comprises adding to an antigen solution 1-45% V/V zinc hydroxide gel or 1-45% V/V iron hydroxide gel and, where appropriate, 1-25% V/V of a lecithin.

## Claims (Claims for the following Contracting State(s): ES, GR)

1. A process for the preparation of an antigen solution containing adjuvant, which comprises adding to an antigen solution 1-45% V/V zinc hydroxide gel or 1-45% V/V iron hydroxide gel and, where appropriate, 1-25% V/V of a lecithin.

## Revendications (Revendications pour l'(les) Etat(s) contractant(s) suivant(s): AT, BE, CH, DE, FR, GB, IT, LI, LU, NL, SE)

1. Utilisation de gel d'hydroxyde de zinc ou de gel d'hydroxyde de fer et éventuellement d'une lécithine pour l'addition en tant qu'adjuvant à une solution d'antigène.

2. Solution d'antigène additionnée d'adjuvant selon la revendication 1, caractérisée en ce que 1 - 45 % v/v de gel d'hydroxyde de zinc ou 1 - 45 % v/v de gel d'hydroxyde de fer et éventuellement 1 - 25 % d'une lécithine ont été ajoutés à la solution d'antigène.

3. Procédé pour la préparation d'une solution d'antigène additionnée d'adjuvant, caractérisé en ce que l'on ajoute à une solution d'antigène 1 - 45 % v/v de gel d'hydroxyde de zinc ou 1 - 45 % v/v de gel d'hydroxyde de fer et éventuellement 1 - 25 % v/v d'une lécithine.

## Revendications (Revendications pour l'(les) Etat(s) contractant(s) suivant(s): ES, GR)

1. Procédé pour la préparation d'une solution d'antigène additionnée d'adjuvant, caractérisé en ce que l'on ajoute à une solution d'antigène 1 - 45 % v/v de gel d'hydroxyde de zinc ou 1 - 45 % v/v de gel d'hydroxyde de fer et éventuellement 1 - 25 % v/v d'une lécithine.
